# EUROPEAN PATENT APPLICATION

(11) **EP 2 875 831 A1**
(43) Date of publication of application: **27.05.2015**
(21) Application number: 14194020.5
(22) Date of filing: 20.11.2014
(51) Int. Cl.: A61K 48/00, C12N 15/87, C12N 15/88, A61K 47/02, A61K 47/20, A61K 47/48, A61K 35/76

(54) **A method of genic transfer into an eukaryotic cell through a viral vector**

(30) Priority: 21.11.2013 IT MI20131944
(71) Applicant: Fondazione I.R.C.C.S. Istituto Neurologico 'Carlo Besta', 20133 Milano (IT)
(72) Inventor: Stellacci, Francesco, 1110 Morges (CH); Pelliccia, Maria, 80013 Casalnuovo Di Napoli (Napoli) (IT); Krol, Silke, 27029 Vigevano (Pavia) (IT)
(74) Representative: Rapisardi, Mariacristina

(57) **Abstract**

A method of transfer of a gene into a eukaryotic cell through a viral vector contained in a solution, comprising a step of engineering the viral vector with the gene encoding for a protein of interest, characterised in that it comprises a following step of improving the transduction efficiency and/or stabilization of the viral vector by interaction of the viral vector with complexing agents of nanometric dimensions and/or agents increasing the viscosity of said solution in which the viral vector is contained, and a following step of infecting the eukaryotic cell with the engineered and complexed viral vector.

## Description

The present invention relates to a new system of transfer of a gene internally of cells based on the use of viral vectors.

The transfer of genetic material internally of the cells is a tool of genetic engineering which includes introduction of an exogenic gene internally of a prokaryotic or eukaryotic cell.

Once internal of the target cell, the exogenic gene is transcribed into RNA before expressing the protein it encodes.

The gene transfer can be applied in various research fields, such as pharmaceutics, the study of micro-organisms, human, plant and animal genetics, agriculture, treatment of genetic diseases and development of both therapeutic or preventative vaccines.

In particular, one of the main applications of gene transfer is gene therapy, which exploits transfer into the cell nucleus of a normal and functioning copy of the mutated gene (and its accessory regulatory elements) through gene transfer vectors.

At present, the vectors employed are essentially of two types: viral and non-viral. Viruses are infecting vehicles *par excellence* and during the course of evolution they have developed mechanisms for inserting their genetic material into the cells of the host.

Consequently, the process of viral infection is exploited for transporting exogenous DNA into cells. Viruses are used as gene transfer vectors, by replacing the essential genes of the viral replication phase with genes of therapeutic interest with the aim of rendering the virus incapable of autonomously reproducing (defective virus) so as to prevent any spread of recombinant viruses.

The present gene transfer techniques have considerable disadvantages among which the possibility of generating new pathogen viruses for recombination with any viruses eventually present in the host, the possibility of insertional mutagenesis (for those which integrate randomly in the genome), inclusion of DNA molecules of limited dimensions in viral vectors, undesired immune reactions, cell toxicity of the molecules or methods used (lipids, polycations, electroporation) and high costs.

Various types of virus are used as vectors of therapeutic genes: among these, one of the most widely used viruses is the adenovirus, which is responsible for infections of the respiratory tract. This is a virus having a linear DNA genome with a double filament enclosed by an icosahedral capsid, having a diameter of 60-90 nm and without a lipid covering.

The adenovirus is used in the field of gene therapy as once it is inside the cell it advantageously does not integrate into the genome and therefore does not create undesired permanent or recombinant mutation problems and effects a transient gene expression. The adenoviral vectors can infect various types of proliferant or non-proliferant mammalian cells. They are easily-manipulable viruses and can contain up to 34 Kb of exogenous DNA, thus enabling insertion of complex gene expression cassettes with genes that can be very long, and various regulatory elements.

One of the disadvantages linked to the use of adenoviruses in the field of gene therapy is the immune response which they can provoke in the host organism, leading to a reduction in the viral quantity caused by inhibitor antibodies.

To date, in the field of gene therapy, efficient insertion and expression at physiological level of the genes in the cells of the organism are still quite difficult to obtain.

A technical task of the present invention is therefore to eliminate the drawbacks linked to the conventional techniques, in particular by providing a new gene transfer method based on the use of viral vectors.

Within the scope of this technical task, an object of the invention has been to provide a gene transfer system that is efficient and stable.

A further object of the invention is to disclose a gene transfer system by viral transduction that is easily applicable in various scopes.

A further object of the invention is to disclose a gene transfer system that enables limiting the doses of virus to be injected.

A still further object of the invention is to disclose a viral transduction system that enables stabilizing and prolonging the conservation of viral vectors in various environments used in the field of vaccination and gene therapy.

A further and not least important object of the invention is to disclose a viral transduction system that is simple and economical.

The technical task, as well as these and other objects, according to the present invention, are attained with a transfer method of a gene into a eukaryotic cell through a viral vector contained in a solution, comprising preparation of the engineered viral vector with the gene encoding a protein of interest for the production of the protein of interest in said eukaryotic cell, characterized in that it comprises complexing agents of nanometric dimensions with said viral vector in order to improve transduction and/or stabilizing efficiency of the viral vector, and in that infects the eukaryotic cell with said engineered and complexed viral vector.

The complexing agents are preferably added in quantities at least sufficient to increase, in a localized manner around the viral vector, the viscosity of the solution in which the viral vector is contained up to a value that increases the stabilization of the viral vector itself.

The method preferably comprises adding viscosizing agents to increase in a uniform manner the viscosity of the solution in which said viral vector is contained.

The viral vector preferably comprises adenovirus, in particular adenovirus serotype 5.

The viral vector preferably comprises picornavirus, in particular Echovirus 1.

The protein of interest preferably comprises green fluorescent protein (GFP).

The complexing agents preferably comprise gold nanoparticles and/or ligands and/or gold nanoparticles coated with ligands.

The viscozing agents preferably comprise glycerol, polyanions, polycations, multivalent ions or glucose.

In particular the ligands comprise MUS, OT, brOT, MPSA, citrate (Auc) or other mixed ligands (hydrophobic/hydrophilic).

The complexing agents advantageously have a negative charge.

The gold nanoparticles preferably have sizes ranging between 3 and 20 nm.

More in particular, the gold nanoparticles coated with said ligands have a diameter of size equal to 2.4±1.2 nm, 4.6±1.5 nm or 20±2.5 nm.

The increase of the transduction efficiency and/or stabilization of the viral vector is realized by means of a short-term incubation phase of said viral vector in the presence of said complexing agents for 1 hour at 37°C.

The increase in the transduction efficiency and/or stabilization of the viral vector is preferably realized by means of a long-term incubation phase of said viral vector in the presence of said complexing agents and viscozing agents, said long-term incubation being realized for up to 15 days at 37°C or between 5-15 days at room temperature.

The present invention further discloses the use of complexing agents having nanometric dimensions comprising gold nanoparticles and/or ligands for improving transduction efficiency and/or stabilization of a viral vector in a transfer method of a gene into a eukaryotic cell with said viral vector.

The present invention lastly discloses a viral vector for transfer of a gene into a eukaryotic cell characterised in that it is incubated with complexing agents having nanometric dimensions comprising gold nanoparticles and/or ligands.

Further characteristics and advantages of the invention will be more fully illustrated in the following description, illustrated by way of non-limiting example with reference to accompanying figures from 1 to 15.

### Improvement in transduction efficiency

Figures 1 and 2 show the level of expression of the eGFP by FACS analysis in the HEK293 cells (Figure 1A) and in the HeLa cells (Figure 1B) infected with the Adenovirus (non-purified) pre-incubated for 1 hour at 37°C with the nanomaterials.
Figure 3 shows the results obtained by confocal microscopy following short-term incubation (1 hour at 37°C) of the non-purified Ad5-eGFP with the MUS:OT AuNP (100 µg/ml) before infection of the zebrafish embryos.
   The embryos infected with the Ad5-eGFP/MUS:OT AuNP mixture show positive GFP cells in the brain, notochord, heart and eyes.
Figure 4 shows the analysis of qRT-PCR for the relative quantification of the GFP levels in the zebrafish embryos. The short-term incubation (1 hour at 37°C) of the eGFP-Ad5 with the nanomaterials (100 µg/ml) i.e. MUS:OT NP, Auc NP, the MUS ligand, and the negatively-charged polymer PPS (sulfonated polystyrene) and the non-electrically charged hydrophilic polymer PEG 8000 (polyethylene glycol with a molecular weight of 8000 dalton).
Figures 5a and 5b show the data obtained by immunohistochemistry analysis in the liver (figure 5b) and the spleen (figure 5a) of the mice treated with Ad5-eGFP with or without pre-treatment with the nanomaterials for 1 hour at 37°C before the injection. Three mice (A, B, C) were used for each experimental condition.
Figures 6a, 6b and 6c report the increase levels of the protein GFP in the spleen of the mice (A, B, C) with respect to "Adenovirus (eGFP-Ad) without heat treatment and without NP" sample.

### Stabilization

Figures 7 and 8 show the results of the 5-day pre-treatment at 37° of the non-purified Ad5-eGFP with the nanoparticles before infecting the HEK293 cells by FACS (figure 7) and qRT-PCR (figure 8).
Figure 9 shows the results obtained by qRT-PCR analysis of the 15-day pre-treatment at room temperature of the non-purified Ad5-eGFP with the nanomaterials before infecting the HEK293 cells.
Figure 10 shows the results obtained by FACS analysis of the 5-day pre-treatment at 37°C of the non-purified Ad5-eGFP with the nanoparticles before infecting the HeLa particles.
Figure 11 reports the long-term treatment in which the non-purified Ad5-eGFP is incubated for 10 days at 37°C with and without nanomaterials before infecting the HeLa cells. The level of expression of the mRNA of the eGFP gene was evaluated using qRT-PCR.
Figure 12 reports the long-term treatment in which the non-purified Ad5-eGFP is incubated for 15 days at room temperature with and without nanomaterials before infecting the HeLa cells.
   The results were obtained by qRT-PCR analysis.
Figure 13 reports the long-term treatment in which the Ad5-eGFP is diluted in PBS and in glycerol 20% and incubated for 15 days at room temperature with and without MUS:OT nanoparticles (100 µg) before infecting the HeLa cells.

### Toxicity

Figure 14 shows the research on the toxicity of the gold nanoparticles in the HeLa cells after 24 hours of incubation through FACS analysis using propidium iodide, an intercalating agent of nucleic acids which is fluorescent in red and which can penetrate only into cells having a compromised plasmatic membrane, so that it is incorporated only by dead cells.
Figure 15 shows the research on immunogenicity of gold particles in pigs. Pigs are very sensitive for immune response to nanoparticles and serve as a model. Lung and cardiac pressure are monitored and often change in response to administrated nanoparticles.

### MATERIALS AND METHODS

### Reagents for synthesis of gold nanoparticles.

The synthesis of the 1-mercapto undecanesulfonate (MUS) ligand was conducted following the procedure reported in a previous work (Verma et al. 2010). The other chemical reagents were supplied by the company Sigma-Aldrich. All the solvents were degassed with nitrogen for 60 minutes immediately before use.

### Synthesis of gold nanoparticles.

For the synthesis of gold nanoparticles (AuNP) functionalized with MUS or MPSA (3-mercapto propanesulfonate acid), 0.9 mmol of gold salt (HAuCl₄) were dissolved in 150 mL of ethanol, and the reaction was maintained under a light flow of nitrogen for the whole duration of the experiment. 0.75 mmol of the molar ratio of interest of the ligands used for the functioning of the nanoparticles (NP) 100MUS (i.e. 100% MUS on the nanoparticles), 66-34MUS:OT (i.e. 66% MUS and 34% OT, octanethiol), 66-34MUS:br-OT (i.e. 66% MUS and 34% brOT, branched octanethiol), and 66-34MPSA:OT (i.e. 66% MPSA and 34% OT) were dissolved in 10-20 mL of methanol and then added to the reaction subjected to shaking.

A saturated solution of sodium borohydride was prepared in 150mL of ethanol, added drop by drop to the gold/thiol mixture. The solution was subjected to shaking for 3 hours under a nitrogen flow in ice and then positioned in a refrigerator for a whole night. The precipitated particles were collected by filtration under vacuum with quantitative filter paper and washed with ethanol, methanol and acetone and dried in a vacuum.

With the aim of removing the free and non-reacted ligands, the precipitate particles were purified by dialysis membranes (MW cut-off of 10,000 Da). The NPs were characterised by transmission electron microscopy and analytical ultracentrifugation.

The 100MUS, 66-34MUS:OT, 66-34MUS:br-OT particles have similar dimensions with a diameter of 4.6 ± 1.5 nm, while the particles coated with MPSA have small dimensions, 2.4 ± 1.2 nm (Carney et al. 2012, Jewell et al. 2011, Astier et al. 2010).

The citrate gold particles (Auc NP) have dimensions of 20 ± 2.5 nm and were synthesized by rapidly adding 1ml of the citrate solution 1% to 5.3 mg di NaAuCl4 x 2H₂O in 25 ml of water Milli-Q (Turkevich, et al. 1951; Sousa et al. 2010).

The solution was heated in the microwave oven for 90 seconds. The concentration of the particles was calculated by UV-Visibile absorption applying the Lambert-Beer law (Liu et al. 2006).

All the nanoparticles employed in the present work show a negative surface electrical charge and the chemical, physical and morphological properties of the nanoparticles and the single ligands are reported in table 1.

The negatively-charged gold nanoparticles have the great advantage of having low toxicity and of improving the stabilizing of the viral vector.

It has on the other hand been demonstrated (data not given here) that gold nanoparticles having a positive surface electrical charge are much more toxic.

The cytotoxicity of the nanoparticles and the ligands was evaluated via the propidium iodide signal by cytometric flow analysis (FACS) and assays of MTT 3-(4,5-Dimethylthiazol-2-yl)-2,5-diphenyltetrazolium bromide.

**Table 1: description of the chemical, physical and morphological characteristics of the nanoparticles used.**

| **Nanoparticles (AuNPs)** | **Surface ligands** | **Dimensions (nm)** | **Potential (mV)** |
|---|---|---|---|
| **66-34 MUS:OT (structured)** | **66% MUS** | **4.5 ± 1.0** | **- 33.1 ± 0.6** |
| **MUS (non-structured)** | **100% MUS** | **4.3 ± 1.2** | **- 38.0 ± 5.3** |
| **66-34 MUS:brOT (non-structured)** | **66% MUS** | **4.3 ± 1.2** | **-31.1 ±0.7** |
| **66-34 MPSA:OT (non-structured)** | **66% MPSA** | **2.4 ± 1.2** | **-30±2** |
| **CITRATE (Auc) (non-structured)** | **100% CITRATO** | **19 ± 2.5** | **-34 ±2** |

### Cells

The HEK293 cells (human embryo kidney cells) and the HeLa cells (human cervix cancer cells) were sourced from the ATCC (American Type Culture Collection, Rockville, MD).

The HEK293 cells were cultivated in DMEM medium (Dulbecco's modified Eagle's medium - Euroclone) containing 10% of bovine foetal serum, FBS SA, (Euroclone), 2mM L-glutamine (Euroclone), and penicillin (100 IU ml ⁻¹)/ streptomycin (100 µgml ⁻¹) (Euroclone). The HeLa cells were maintained in MEM-glutamax (Invitrogen) medium added-to with 10% of FBS SA, 1% of non-essential amino acids (Euroclone), 1% of pyruvate sodium (Euroclone) and penicillin and streptomycin (Euroclone). The cell cultures were maintained at 37°C in moist atmosphere (5% CO2, 95% air).

### Adenoviral vector expressing eGFP

The present work involved the use of human adenovirus serotype 5 (Ad5) with genes E1- and-E3 deleted and engineered with the gene expressing the protein eGFP (enhanced green fluorescent protein) under the transcription control of the constitutively active promoter cytomegalovirus (CMV). The recombinant vector Ad5-eGFP is generated following the AdEasy Vector™ system protocol (He et al.1998).

The adenovirus was amplified through successive amplification cycles in the HEK293 cells in DMEM 2% (Graham et al.1977). The cells were infected for 48/72 hours and with the maximum cytopathic effect (about 80% of CPE) were collected and lysed through three consecutive freezing/thawing cycles with the aim of releasing the new viral progeny. The supernatant containing the adenovirus was isolated by centrifuging at 2000g for 20 min, aliquoted, titrated and conserved at -80°C (Graham et al. 1977, He et al.1998, Harrison et al.1977).

The non-purified adenovirus, being directly contained in the culture medium once harvested from the HEK293 cells, was titrated by flow cytometry (FACS) in the HeLa cells with the aim of quantifying the percentage of positive GFP cells after 48 hours from the infection. The titre of the adenoviral vector is 1.3 x 10⁵ TU/ml (transducing units per ml). The *in vitro* experiments were carried out using non-purified Ad-eGFP, where the virus is directly in the culture medium, and with purified Ad-eGFP.

The purified Ad-eGFP was supplied by Vector BioLabs and the virus is purified from the cell culture by centrifuging on a gradient of caesium chloride and conserved in PBS with 10% glycerol at -80°C. The purified Ad-eGFP has a titre of 1.1 x10¹¹ IFU/ml (infecting units per ml). The *in vivo* studies on zebrafish embryos were carried out with the non-purified virus, while the experiments conducted on mice included the use of the purified adenovirus.

### Transduction with Adenovirus in vitro

### Pre-treatment of adenovirus-eGFP with the NPs and the ligand.

The HeLa and HEK293 cells were seeded in 12-well dishes respectively in number of 6 x 10⁴ and 1 x 10⁵ cells per 1ml per well in culture media containing FBS 10%.

The following day the medium was replaced with 2% medium before proceeding with the infection of the virus.

In the present work, Ad5-eGFP was subjected to two types of incubation with the nanoparticles and ligand, short-term pre-treatment and long-term pre-treatment, before infecting the target cells.

The short-term pre-treatment of the vector Ad5-eGFP with the nanoparticles and the ligand includes a step of incubation for 1 hour at 37°C before the infection. In this experimental condition the HEK293 cells were infected with the non-purified Ad5-eGFP at a multiplicity of infection (MOI) of 0.001, while the infection of the HeLa cells was carried out at a MOI of 0.1 for the non-purified virus and at a MOI of 125 for the purified Ad.

Two different MOIs were used for the two cell lines since the infectivity of the adenovirus is less pronounced in the HeLa cells with respect to the HEK293 cells. Further, two virus preparations were used, purified and non-purified, with two different MOIs in the HeLa cells since the non-purified virus has a much lower titre than the purified Ad and is characterised by the presence of proteins and some cellular detritus contained in the culture medium of the cells from which the virus is harvested.

The long-term pre-treatment, on the other hand, includes 2 types of incubation, the first consisting in incubating the virus and NP or ligands mixture for 3, 5, 10, or 15 days at 37°C while in the second condition the virus-NP and virus-ligand mixture was incubated for 5-15 days at room temperature before infecting the cells. For the long-term pre-treatment, the HeLa cells were infected at a MOI of 0.1, while the HEK293 cells were infected with a MOI of 0.01.

The Ad/NP and Ad/ligand mixtures previously incubated for the long-term pre-treatment were added to the cells. The infection was evaluated after 48 hours by epifluorescence microscopy, FACS analysis in order to quantify the percentage of transduced cells which express the GFP and quantitative RT-PCR (qRT-PCR) so as to determine the number of copies of the mRNA of the GFP gene. For each experimental condition the experiments were conducted in duplicate or triplicate.

With the aim of carrying out the FACS analysis, the cells were collected, washed with PBS (a saline phosphate buffer) and fixed with paraformaldehyde 1%. The FACS analysis was carried out using the FACS CANTOII cytofluorimeter (Beckton Dickinson).

For the qRT-PCR analysis, the total RNA was extracted and purified from the cells using the RNAeasy Mini Kit (Qiagen). The quantity of extracted RNA was determined by a spectrophotometric reading (NanoDrop® ND-1000). The mRNA was retro-transcribed into cDNA with SuperScript VILO cDNA Synthesis Kit (Invitrogen) using random primers according to the manufacturer's indications. The cDNA (5 ng) was amplified in triplicate in a reaction volume of 10 µl using 5 µl of TaqMan PCR Mastermix 2x No UNG (Applied Biosystems, Foster City, CA) and 0.5 µl of TaqMan Gene expression assay 20x (Applied Biosystems, Foster City, CA).

The qRT-PCR reaction was carried out in the ABI/Prism 7900 HT Sequence Detector System (Applied Biosystems) thermocycler, applying a pre-PCR step of 10 min at 95°C, followed by 40 cycles of 15 s at 95°C and 60 s at 60°C.

For the negative control, samples containing the RNA template were used but without the reverse transcriptase enzyme.

For each sample, the level of expression of the GFP was normalized with respect to the housekeeping gene (constitutively expressed reference gene) of the β-actin by means of the comparative Ct (Cycle-threshold) method.

The level of the target gene (eGFP) is expressed via a relative quantification based on the control sample defined as the calibrator which becomes the sample 1x, and the other samples are reported as an increase of the number of copies of mRNA with respect to the calibrator.

### Transduction with Adenovirus-eGFP in vivo in Zebrafish embryos.

### Lines of Zebrafish, maintenance and short-term incubation of Ad- eGFP with the nanoparticles and ligands.

The strain of Zebrafish (Zf, *danio rerio)* was raised and coupled following conventional conditions. In the present work the wild-type AB Zf line and the mutant line *mitfa* was used. The fish were maintained in respect of the EU rules relating to laboratory animals. During the late step of gastrula the Zf embryos were dechorionated with a diluted solution of Pronase (2 mg/ml in *fish water).*

With the aim of removing the enzyme, the embryos were washed 3-4 times with *fish water.* Then the dechorionated embryos were distributed in a plate having 12 or 24 wells, in groups of 8-10 embryos per well in a volume of 1 ml of *fish water* (E3 buffer, containing 5 mM of NaCl, 0.17 mM of KCl, 0.33 mM of CaCl2 and 0.33 mM of MgSO4).

The embryos were infected with Ad5- eGFP (1.3x10⁴ virus per well containing 8-10 embryos) and the mixtures of Ad5-eGFP/NP and Ad-eGFP/ligand previously incubated for 1 hour at 37°C following the protocol for short-term treatment described for the *in vitro* infection of the HeLa cells. The zebrafish were incubated at 30°C up to the fourth day of embryo development. Each experimental condition was conducted in triplicate.

The day following the infection the water was replaced with a new preparation of *fish water* without renewing the infection with the Ad5.

After 48 hours from the infection, the embryos were observed using fluorescence stereomicroscopy connected to a high-resolution digital camera (Nikon) with the object of evaluating the presence of fluorescent cells expressing the GFP.

After 4 days from the infection the embryos were subjected to immunofluorescence assays and observed using the confocal microscope (TCS SP2 Leica confocal microscope using the 20x and 40x water-immersed lens and the 488 nm laser).

This type of analysis provided qualitative information, showing only the zebrafish cells having a high GFP signal.

With the aim of effecting a quantitative study of the levels of expression of the eGFP gene, assays of quantitative RT-PCR (qRT-PCR) were made.

For the analyses of qRT-PCR, the total RNA of the zebrafish was extracted on the fourth day of embryo development and viral infection using the TRIzol (Gibco) reagent. The RNA was purified with the DNase I and quantified by spectrophotometric reading. The mRNA was reverse-transcribed into cDNA with the Superscript III Reverse Transcriptase kit (Invitrogen) kit, using random primers. The levels of the eGFP gene were quantified by qRT-PCR using the gene of the β-actin as an internal control as previously described for the qRT-PCR analyses carried out in the *in vitro* experiments.

### Gene transduction with Adenovirus in vivo in mice.

The mice were housed in standard ambient conditions of sterility. All the procedures applied were approved by the local Institutional Animal Care and Use Committees and conducted in conformity with the local regulations and the provisions of the European Union, nos. 86 and 609. The present study used wild-type male mice, eight weeks old, intravenously injected in the tail with pre-treated purified Ad5-eGFP (1.1x10⁹ ifu, infective units) and not with the NPs (MUS:OT, MUS, MUS:brOT, 250µg each NP) the ligand MUS (37.5 µg) for 1 hour at 37° before carrying out the injections. For each experimental condition 3 mice (mice a, b and c) were used.

In parallel, to evaluate any eventual toxicity linked to the nanomaterials, the NPs and the ligand, with no addition of virus, were injected into the mice.

After 5 days from infection the animals were sacrificed and the tissues, such as liver, spleen, lungs, kidneys, heart and brain were removed and fixed in 4% paraformaldehyde in order to carry out the immunohistological analysis with the aim of evaluating the presence of cells expressing the GFP.

The tissues were preserved in paraffin and cut into serial sections of 3µm using the Leica ASP 300 processor. The paraffin was then removed using xylene and the sections were rehydrated by scale of alcohols.

The treatment for the recuperation of the antigen (antigen retrieval) was performed by immersing the tissues in a preheated retrieving citrate buffer solution pH 6, followed by blocking nonspecific sites by FBS for 90 minutes.

The incubation with the primary antibody for GFP (diluted 1:100, Santa Cruz) was carried out for a whole night, followed by incubation with the biotinylated secondary antibody (1:200 Vector Lab) for 1 hour.

The immunolocalization reaction was detected by horseradish peroxidase (HRP), using the Vectastain Elite Avidin-Biotin Complex-Peroxidase kit, followed by the development of the chromogen substrate (Peroxidase substrate kit, DAB, SK-4100; Vector Lab). The sections were counter-stained with haematoxylin (Mayer) and viewed by bright-field microscope.

### Results and discussion

**I. Increase of the eGFP gene expression by short-term pre-treatment of the adenovirus with *in vitro* nanomaterials;**
**II. Stabilization of the adenovirus by long-term treatment with nanomaterials by *in vitro* experiments.**
**III.Non-toxicity of the nanomaterials used**

The serotype 5 adenovirus engineered for the expression of the eGFP gene was pre-incubated for 1 hour and 3-5-10-15 days at 37°C and 5-15 days at room temperature with the ligand and the gold nanoparticles having different surface functionalizations and dimensions.

The Ad5-eGFP/ NP and Ad5- eGFP/ ligand mixture was added to the HeLa and HEK293 cells. After 48 hours from the infection the fluorescence signal of the GFP and the level of gene expression coming from the cells transduced by the adenovirus was compared with the non-infected cells with the virus by FACS and qRT-PCR analysis.

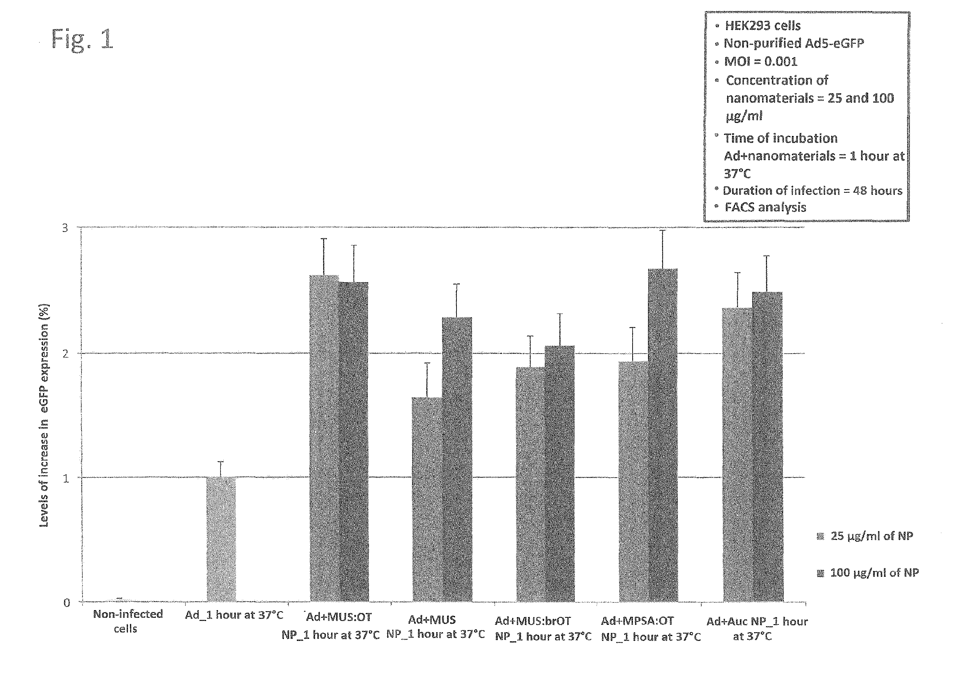

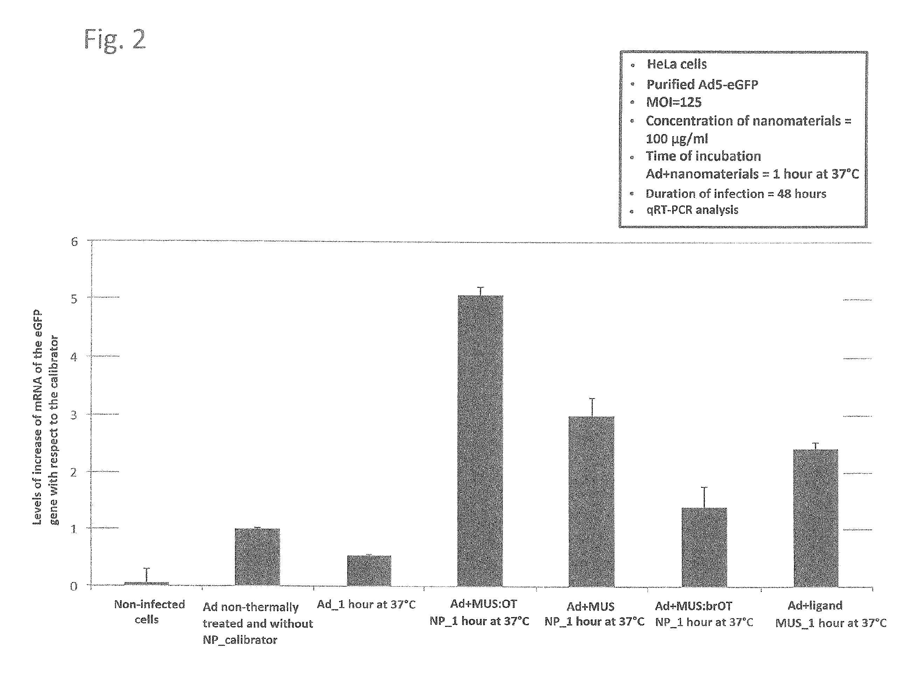

In figures 1 and 2 the *in vitro* data is reported, relating to the pre-treatment of the virus for 1 hour at 37°C with the nanomaterials before infecting the HEK293 and HeLa cells.

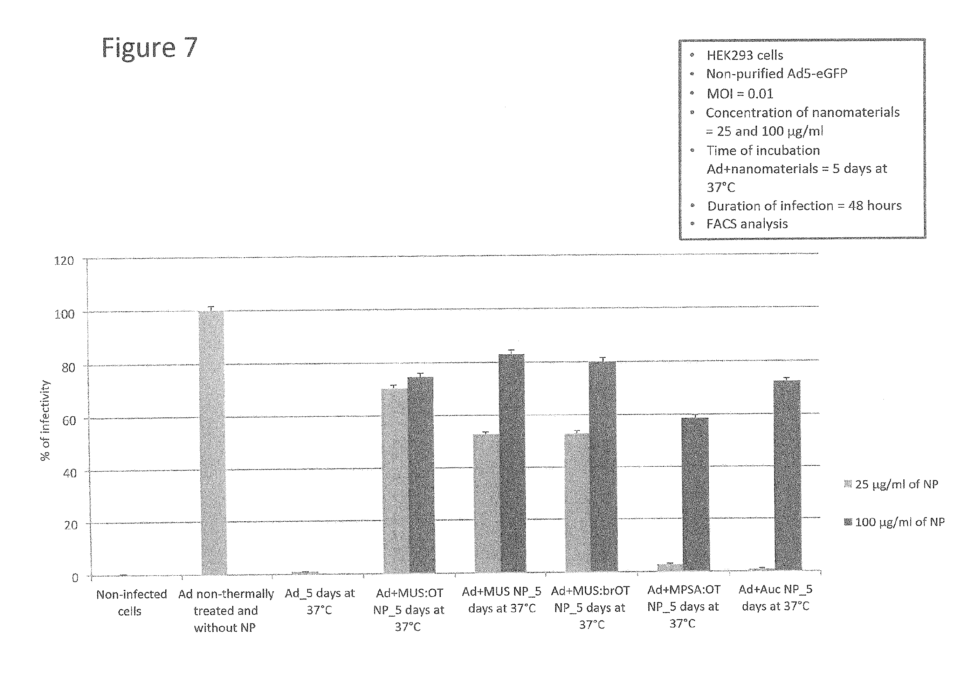

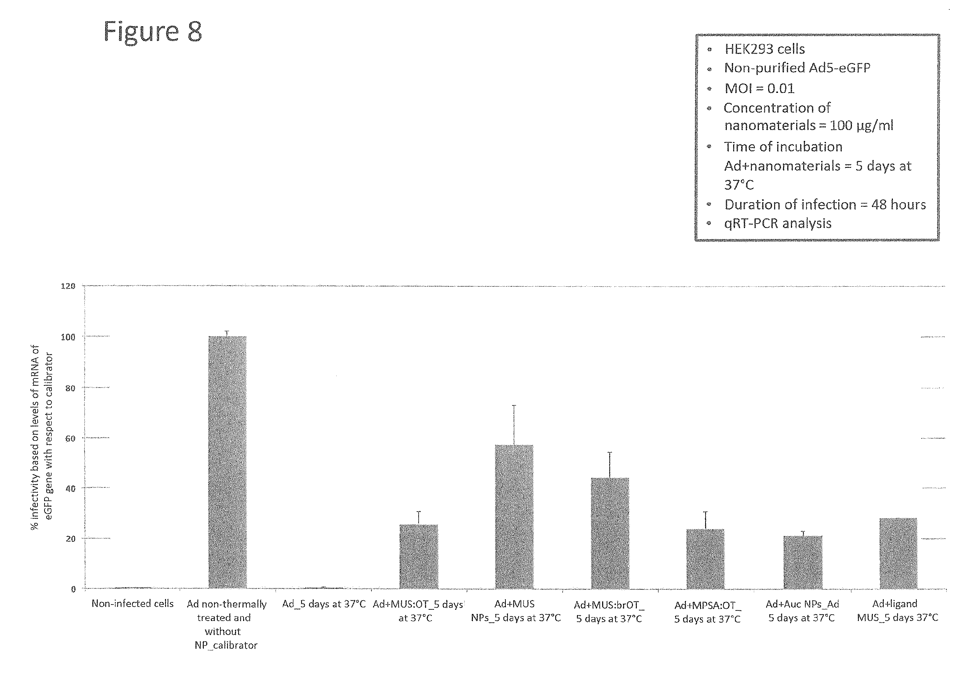

Figures 7 and 8 report the pre-treatment of the Ad5 for 5 days at 37°C before infecting the HEK293 cells.

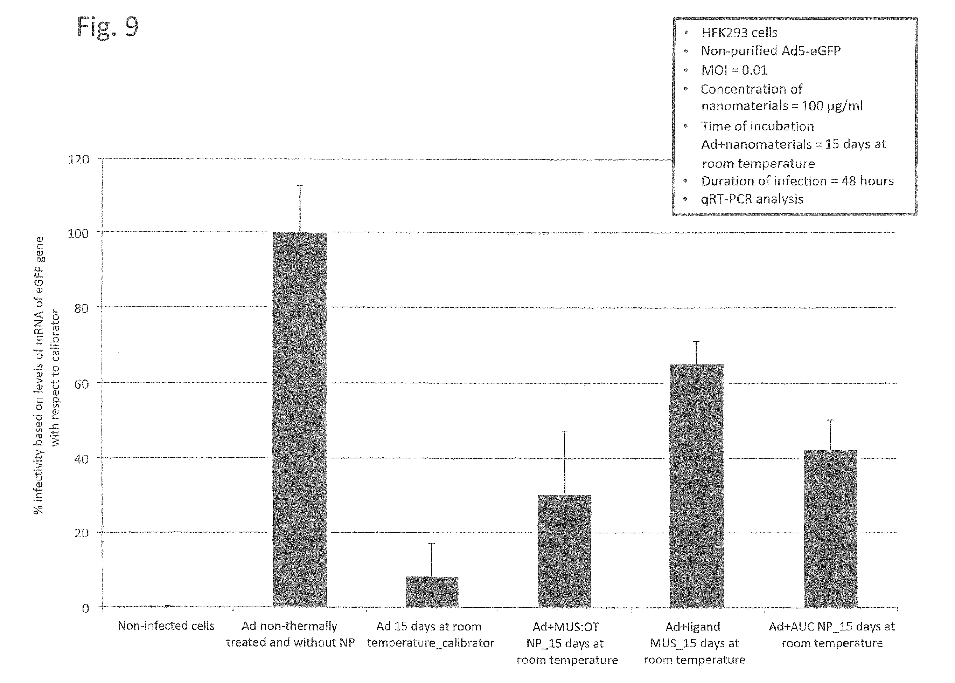

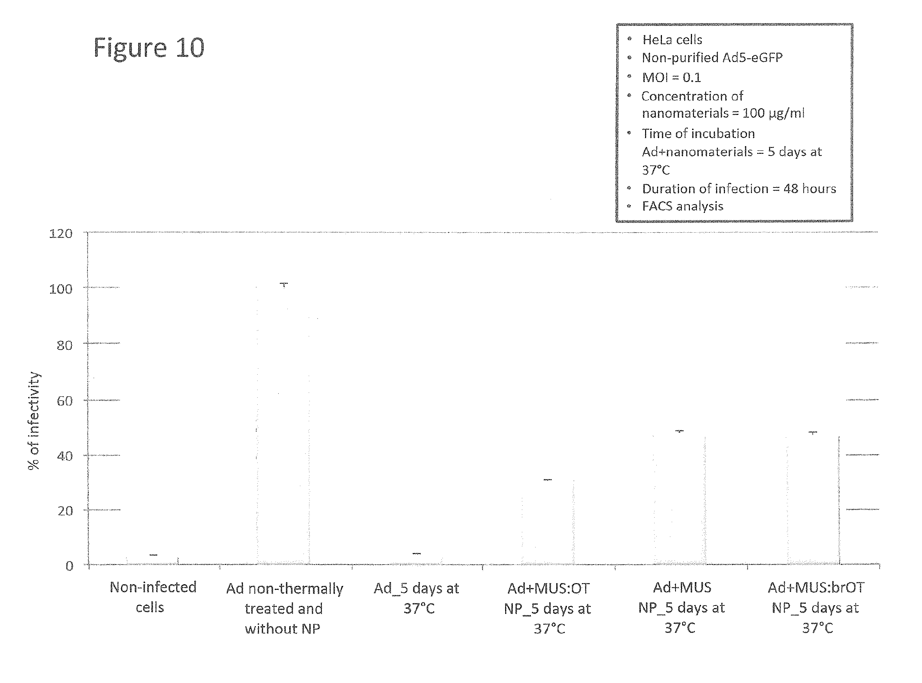

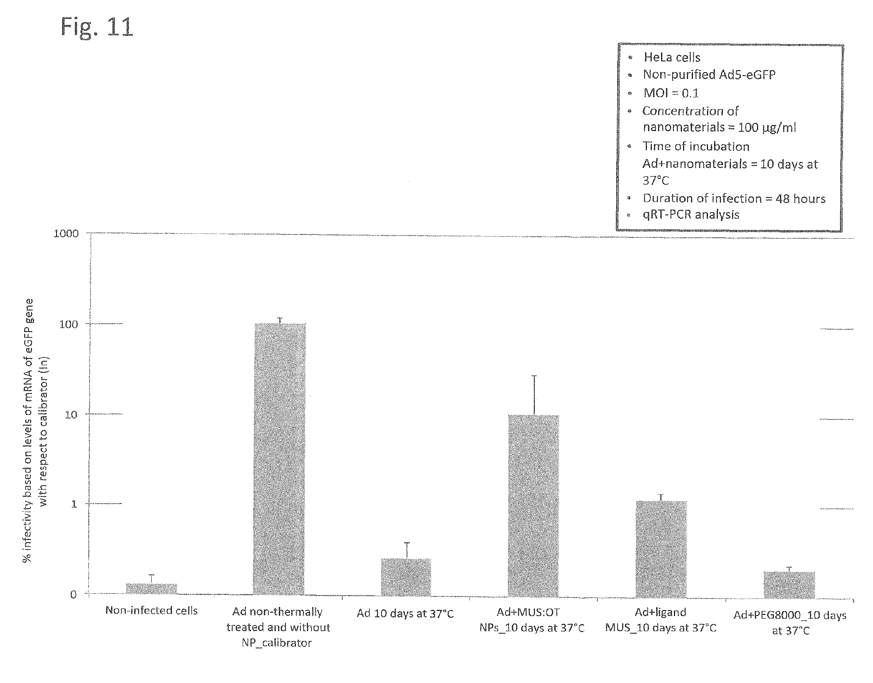

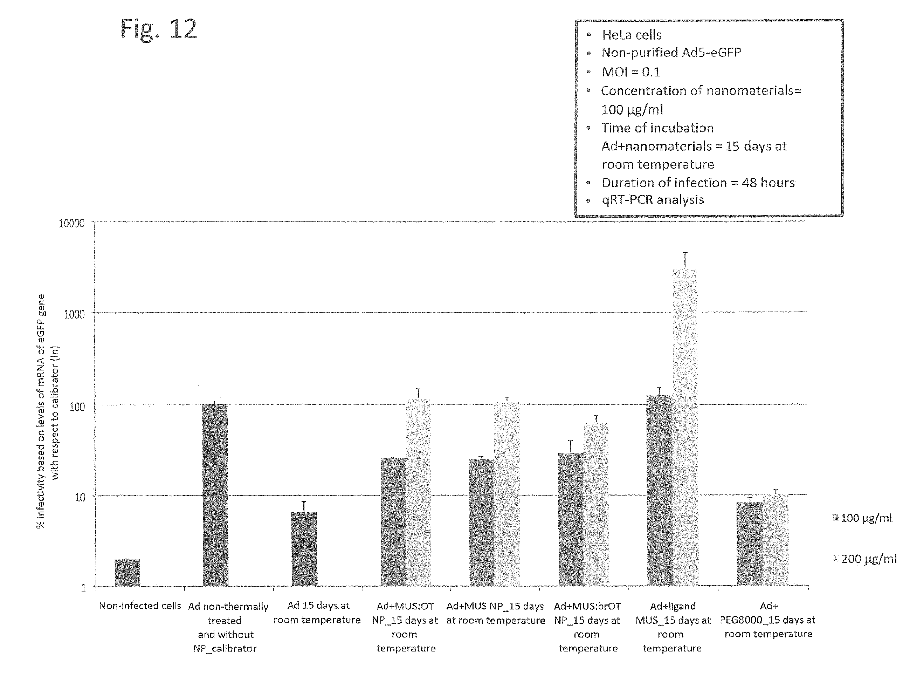

Figure 9 reports the results of pre-treatment of the Ad5 for 15 days at room temperature before the infection in the HEK293 cells. Figure 10 shows the results of the pre-treatment of the Ad5 for 5 days at 37°C before infecting the HeLa cells. Figure 11 reports the pre-treatment of Ad5 for 10 days at 37°C while figure 12 shows the results relative to the pre-treatment of the Ad5 for 15 days at room temperature before infecting the HeLa cells.

In the graphs of figures 1 and 7, the columns coloured in grey indicate the treated virus with 25 µg/ml of NP, while the respective darker colours (black) indicate the virus treated with 100 µg/ml of nanoparticles. The graph of figure 1 represents the virus-NP pre-treatment for 1 hour at 37°C (increase in the expression of the GFP gene), while the graph of figure 7 relates to the pre-treatment of the virus with the NP for 5 days at 37°C before infecting the cells (stabilization of the virus in adverse conditions).

The results illustrated in figures 1 and 2 are extremely interesting and evidence the fact that the cells infected with the NP-pre-treated adenovirus for 1 hour at 37°C has a level of expression of the GFP that is significantly higher with respect to the "adenovirus non- incubated with NP" control.

The data relating to the pre-treatment for increasing the stability of the long-term adenovirus with the NPs (figures 9, 10, 11, 12 e 13) are very promising. In this experimental condition, the non-NP-pre-treated adenovirus almost completely loses its ability to infect the cells and thus to express the GFP protein. Instead, the adenoviral vector incubated with the NPs maintains its infectivity, expressing a good level of GFP in the target cells with respect to the positive control, in which the virus has been directly added to the target cells with no heat treatment and without addition of the nanomaterials (stabilization of the virus in adverse conditions).

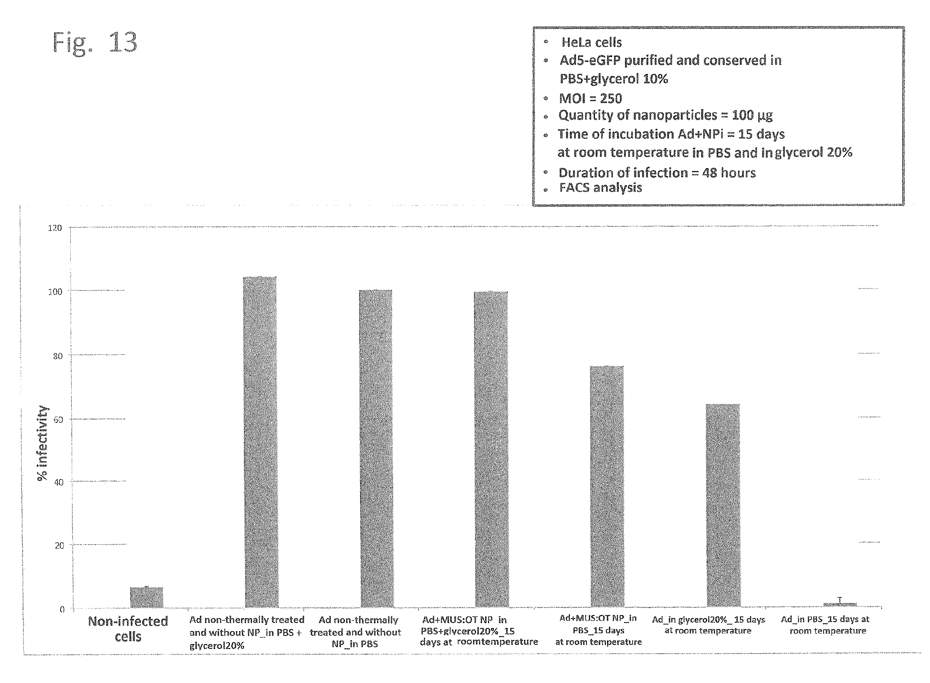

These results clearly show that the virus incubated with the nanomaterials for 3-5-10-15 days at 37°C and 5-15 days at room temperature maintains its infectivity with regard to the cells. Further experimental data (reported in figure 13) show the long-term treatment in which the Ad5-eGFP is diluted in PBS and in glycerol 20% and incubated for 15 days at room temperature with and without MUS:OT nanoparticles (100 µg) before infecting the HeLa cells.

The Ad diluted in PBS and incubated alone for 15 days at room temperature almost totally loses its capacity to infect the cells. The Ad diluted in glycerol 20% and incubated for that amount of time conserves its infectivity as the cells express a good GFP level. This study shows that the glycerol 20% is able to preserve the stability of the virus. In the same way the Ad incubated for 15 days at room temperature with the NPs in PBS or in glycerol 20% is stabilized and is able to induce a good level of infection. The error bar in figure 13 represents the average of the values ± standard deviation (n>2).

The addition of the glycerol 20% changes the viscosity of the whole solution in which the viral vector is contained. The nanoparticles are instead able to change the viscosity of the micro-ambient close to the viral vector. The changing of the viscosity increases the stabilization of the viral vector which following incubation in the presence of glycerol 20% or in the presence of the nanoparticles is able to induce a good level of infection.

### Increase in the expression of the eGFP gene through short-term pre-treatment of the Adenovirus with nanomaterials in vivo.

### Zebrafish

The present work includes a study of the interaction between the functionalized nanoparticles on the surface and negatively charged and the MUS ligand with the adenovirus with the aim of increasing the transduction and the stability of the adenovirus and therefore increasing the efficiency thereof as a viral vector for gene therapy treatment and in the field of vaccination. Two experimental conditions were tested which related to the interaction between the Ad and the nanomaterials: 1) short-term incubation of the Ad5-eGFP with the nanomaterials for 1 hour at 37°C before infecting the cells, the zebrafish embryos and the mice; 2) long-term incubation of the eGFP-Ad with the NP and the ligand before infecting the cells.

In the experiments conducted *in vitro* the short-term treatment of the Ad5-eGFP with the nanomaterials induces an increase in the levels of expression of the transgene GFP of 2-5 times greater with respect to the non-treated virus as evidenced by FACS and qRT-PCR analysis and in figures 1, 2 and 3.

The results were successfully confirmed with success with MUS:OT NP by means of *in vivo* experiments based on the use of zebrafish embryos *(Danio rerio).* Petrella et al. 2002 identified, in the zebrafish, a homologue of the coxsackie and adenovirus receptor (CAR), the prime receptor for the ingress of the adenovirus into the cells.

The CAR receptor has, in man (hCAR) and zebrafish (zCAR), 52% amino acid similarity along the whole chain of the protein and 66% identity in the cytoplasmic domain. During the zebrafish embryo development, the CAR receptor is expressed in various tissues, including the kidneys, skin, heart, liver, retina and the central nervous system.

There is no evidence in the literature that the zCAR functions naturally as a receptor of the adenovirus and to date there have been no descriptions of infections by the adenovirus in zebrafish (Petrella *et al.*2002, Raschperger *et al.*2008).

These observations led to the use of the zebrafish as the alternative vertebrate model system for evaluating the stabilizing effect and increasing the mediated viral transduction from the nanoparticles onto the human adenovirus.

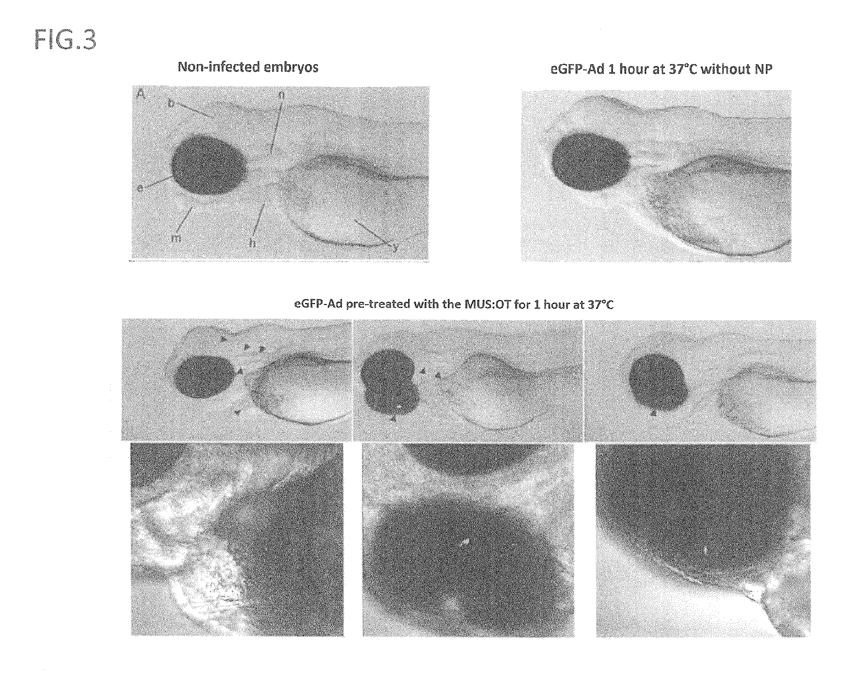

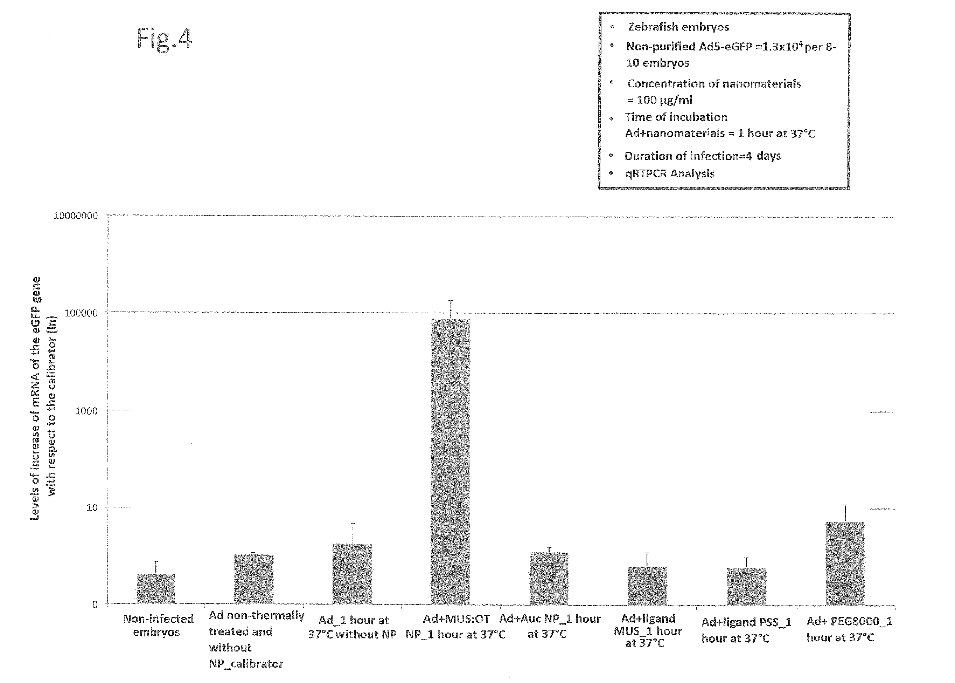

The zebrafish embryos were infected with the non-purified pre-treated adenovirus and not with the nanomaterials for 1 hour at 37°C. The confocal microscopy analysis in figure 3 and the qRT-PCR in figure 4 demonstrate that the pre-treated adenovirus pre-treated for 1 hour at 37°C with the MUS:OT nanoparticles shows a level of expression of the GFP that is significantly greater than the embryos infected with the adenovirus not pre-treated with the NPs. No increase of expression of the GFP levels was encountered for the Ad treated with the Auc NP and the MUS ligand.

### Mice

The pre-treatment of the Ad5-eGFP with the nanoparticles (nanoparticles MUS:OT, MUS, MUS:brOT and the MUS ligand) for 1 hour at 37°C before the injection in the mice demonstrated an important infection of the adenovirus in the liver, both in the hepatocytes and in the Kupffer cells, as well as in the spleen at the marginal zone of the follicle.

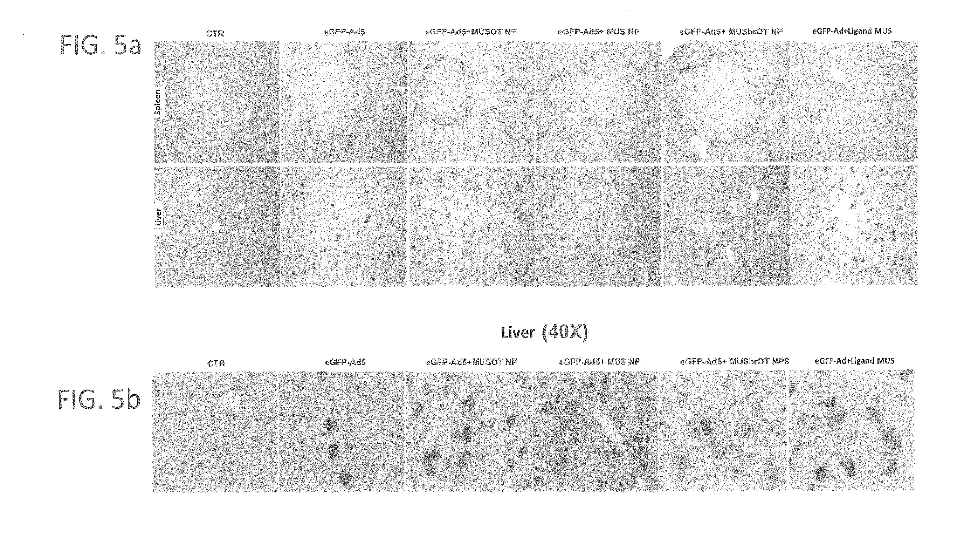

Figure 5a and 5b report the expression of the positive GFP cells in the liver and in the spleen of mice by immunohistochemical analysis. The mice infected with the Ad5 pre-treated with the nanomaterials show a higher GFP expression level (cells with the brown signal indicate the result of the recognition reaction of the antibody in comparison to the GFP protein expressed in the tissues) with respect to the mice infected only with the virus (Ad5-eGFP) with no thermal pre-treatment and with no incubation with the nanomaterials.

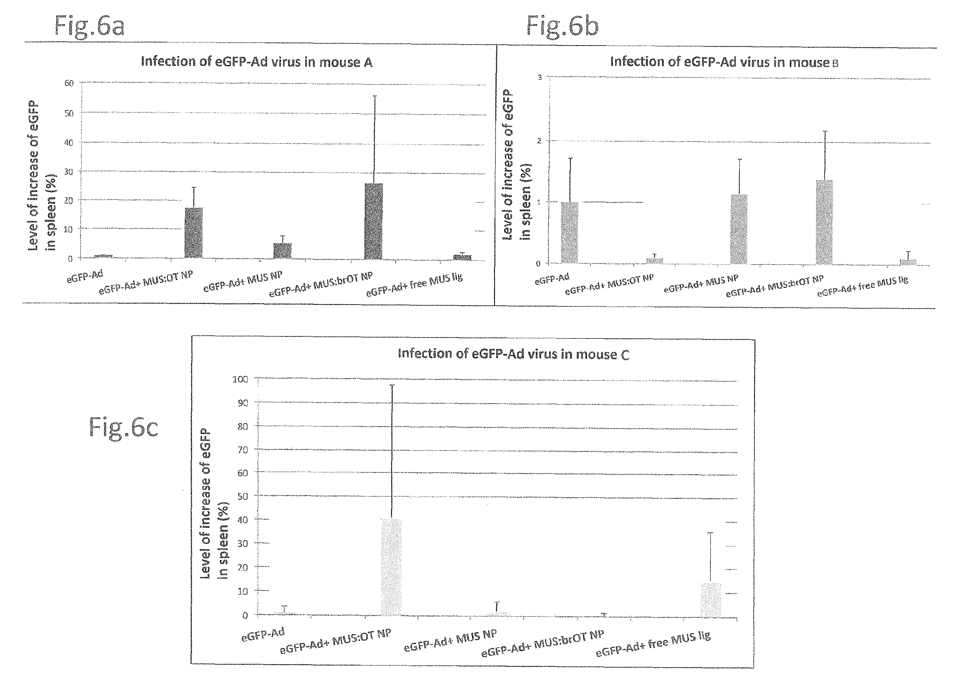

Figures 6a, 6b and 6c report the relative quantification of the GFP expression in the mice spleen with respect to the Adenovirus (with no thermal treatment and without NP) sample in the three animal groups (mice A,B,C). The quantification of the GFP signals was effected by using imageJ software on the basis of the immunohistochemical analyses. The results indicate that the mice infected with the Ad5-eGFP pretreated with the nanomaterials show a GFP expression level that is 2-20 times greater than the control (adenovirus without thermal treatment and without nanomaterials).

The ability of the nanomaterials to induce an increase in the gene expression of the adenovirus is successful, and also demonstrated in mice.

The animals injected only with nanoparticles and ligand (no virus) had no morphological damage in the analyzed tissues with respect to the control not treated with virus and nanomaterials.

The encouraging preliminary results obtained in mice mean that the nanomaterials of the present invention can be proposed as complexing agents with the adenovirus, with no covalent link, with the aim of improving and increasing the efficiency of the gene transfer based on the adenoviral vector and minimizing the *in vivo* collateral effects due to the administration of the virus, reducing the doses of Ad to be injected. Further, by reducing the quantity of adenoviral vector with the use of the nanomaterials, in theory the toxicity can be attenuated by reduction of the production of the neutralizing Ab against the adenovirus.

### Nanotoxicology

With the aim of evaluation the eventual toxicity of the nanoparticles, cell vitality was determined in the HeLa cells following 24 hours of incubation by FACS analysis, using propidium iodine (PI) with the aim of discriminating the dead cells.

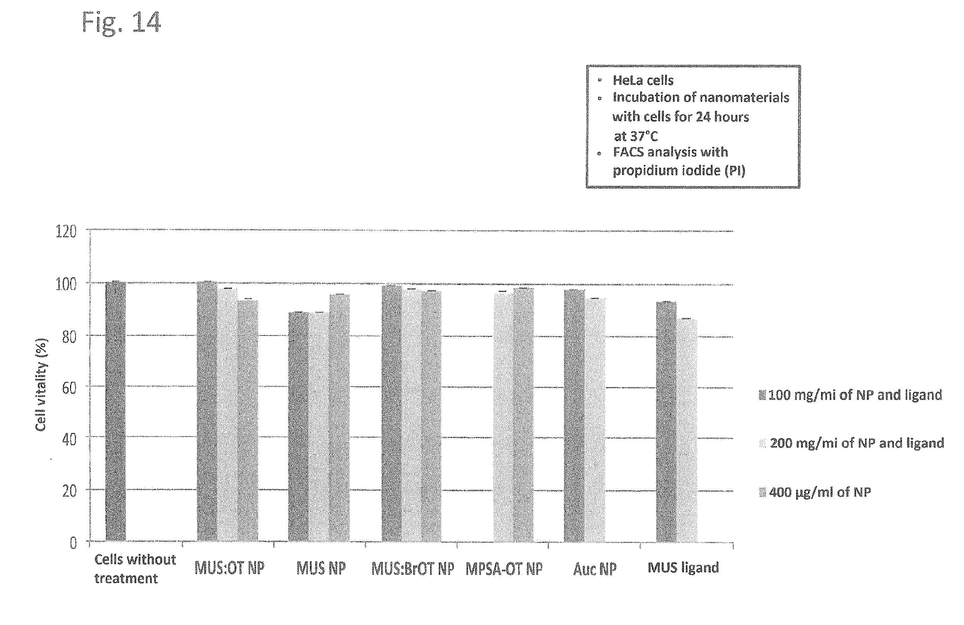

As reported in figure 14, only the NPs fully functionalized with the MUS molecule show a slight cytotoxicity. The other NPs and the ligand exhibit an insignificant toxicity with respect to the HeLa cells for all the tested concentrations (100 e 200 µg/ml). This data is fundamental in relation to nanotoxicology as one of the main limits connected to the use of nanomaterials in the biomedical field regards the toxicity of the material itself.

Further, the experiments conducted in vivo on the zebrafish embryos and mice where the animals were treated only with the nanomaterials (without virus) did not exhibit any toxicity or death due to the presence of the nanomaterials in comparison with the negative control (sample not treated with virus and nanomaterials).

### Immunotoxicity

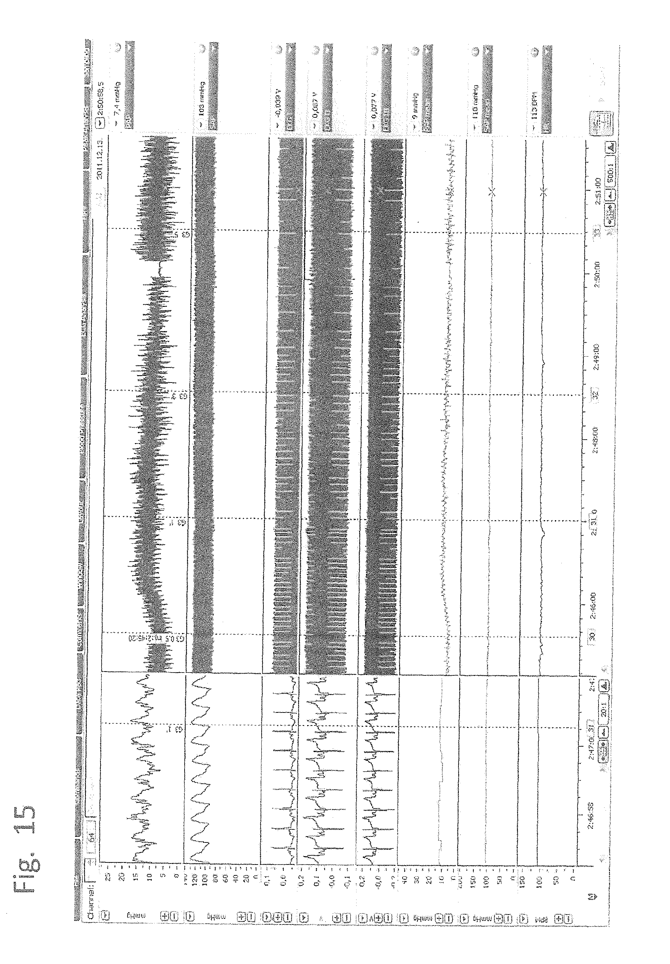

In the ambit of toxicity linked to nanomaterials it is well known that the nanoparticles can cause an immunological response which sometimes can lead to the death of the organism. With the aim of evaluating this type of toxicity, experiments with pigs were conducted; pigs are extremely sensitive to the immunological response induced by nanoparticles. Three pigs weighing 20-30 Kg were injected with growing concentrations of nanoparticles up to 0.5mg/kg of body weight with the aim of monitoring the cardio-pulmonary response. Only the highest concentration of nanoparticles generated a slight reaction characterised by an increase in the pulmonary pressure. This effect was shown to be completely reversible after 1 minute (figure 15).

In conclusion the nanoparticles coated with a monostrate of ligands or the ligand alone have an effect of increasing on the serotype 5 adenovirus-mediated gene transduction after pre-treatment with the virus for a short time (1 hour at 37°C) and a stabilizing effect on the virus by incubation for long times at 37°C (3-5-10-15 days) and at room temperature (5-15 days).

Both gene transfer and viral vector stabilization are advantageously increased also by the use of viscozing agents which increase the viscosity of the solution in which the viral vector is contained.

The same behaviour of the nanomaterials has advantageously also been observed for two picornaviruses: ECHO-virus-1 (Enteric Cytopathic Human Orphan; potential vaccines) and *Theiler*'*s* Murine Encephalomyelitis *Virus* (experimental model for multiple sclerosis) (data not shown).

The complexing agents of the present work and in particular the gold particles coated with ligands emphasized their ability in stabilizing and protecting the viral vectors in adverse conditions, during which an inactivation mechanism of the virus is in theory exhibited following the denaturizing of the proteins of the capsid. Consequently the interaction of the adenoviral vector with the gold nanoparticles facilitates the maintaining of the infective capacities, ensuring a good level of transfer of the gene of interest.

In the light of the data obtained it can be sustained that the gene transfer system of the present invention can function with equal success with other viral particles without lipid covering, such as the adenovirus and the picornaviruses, and engineered for transfer of another type of gene.

## Claims

1. Method of transfer of a gene into an eukaryotic cell through a viral vector contained in a solution, comprising the preparation of the genetically engineered viral vector with a gene encoding for a protein of interest for the production of said protein of interest in said eukaryotic cell, **characterized by** making interact complexing agents of nanometric dimensions with said viral vector to improve transduction efficacy and/or stabilization of the said viral vector and by infecting said eukaryotic cell with said engineered and complexed viral vector.

2. Method of transfer of a gene into an eukaryotic cell through a viral vector according to claim 1, **characterized in that** said complexing agents are added in a quantity at least sufficient to increase in a localized manner around the viral vector the viscosity of the solution in which the viral vector is contained up to a value that increases the stabilization of the viral vector itself.

3. Method of transfer of a gene into an eukaryotic cell through a viral vector according to claims 1 and 2, **characterized by** adding viscosizing agents to increase in a uniform manner the viscosity of the solution in which said viral vector is contained.

4. Method of transfer of a gene into an eukaryotic cell through a viral vector according to any of the preceding claims, **characterized in that** said viral vector comprises adenovirus, in particular adenovirus serotype 5 or enterovirus, in particular Echovirus-1.

5. Method of transfer of a gene into an eukaryotic cell through a viral vector according to any of the preceding claims, **characterized in that** said protein of interest comprises green fluorescent protein (GFP).

6. Method of transfer of a gene into an eukaryotic cell through a viral vector according to any of the preceding claims, **characterized in that** said complexing agents comprise gold nanoparticles and/or ligands and/or gold nanoparticles coated with ligands.

7. Method of transfer of a gene into an eukaryotic cell through a viral vector according to any of the preceding claims, **characterized in that** said viscozing agents comprise glycerol, polyanions, polycations, multivalent ions or glucose.

8. Method of transfer of a gene into an eukaryotic cell through a viral vector according to claim 6, **characterized in that** said ligands comprise MUS, OT, brOT, MPSA, citrate (Auc) or other mixed ligands (hydrophobic/hydrophilic).

9. Method of transfer of a gene into an eukaryotic cell through a viral vector according to any of the preceding claims, **characterized in that** said complexing agents have a negative charge.

10. Method of transfer of a gene into an eukaryotic cell through a viral vector according to claim 6, **characterized in that** said gold nanoparticles have sizes ranging between 3 and 20 nm.

11. Method of transfer of a gene into an eukaryotic cell through a viral vector according to claim 6, **characterized in that** said gold nanoparticles coated with said ligands have a diameter of size equal to 2.4±1.2 nm, 4.6±1.5 nm or 20±2.5 nm.

12. Method of transfer of a gene into an eukaryotic cell through a viral vector according to any of the preceding claims, **characterized in that** said increase of the transduction efficiency and/or stabilization of the viral vector is realized by means of a short-term incubation phase of said viral vector in the presence of said complexing agents for 1 h at 37°C.

13. Method of transfer of a gene into an eukaryotic cell through a viral vector according to any of the preceding claims 1 to 11, **characterized in that** said increase in the transduction efficiency and/or stabilization of the viral vector which is realized by means of a long-term incubation phase of said viral vector in the presence of said complexing agents and viscozing agents, said long-term incubation being realized up to 15 days at 37°C.

14. Method of transfer of a gene into an eukaryotic cell through a viral vector according to any of the preceding claims 1 to 11, **characterized in that** said increase in the transduction efficiency and/or stabilization of the viral vector is realized by means of a long-term incubation phase of said viral vector in the presence of said complexing agents and said viscozing agents for 5 to 15 days at room temperature.

15. Use of complexing agents of nanometric dimensions comprising gold nanoparticles and/or ligands to increase the transduction efficiency and/or stabilization of a viral vector in a method of transfer of a gene into an eukaryotic cell through said viral vector according to any of claims 1 to 14.

16. Viral vector for the transfer of a gene into an eukaryotic cell **characterized in that** it is incubated with complexing agents of nanometric dimensions comprising gold nanoparticles and/or ligands to increase its transduction efficiency and/or its stabilization according to any of claims 1 to 15.
